# FASCICULE DE BREVET EUROPEEN

(11) **EP 2 718 686 B1**
(45) Date de publication et mention de la délivrance du brevet: **18.09.2019**
(21) Numéro de dépôt: 12729446.0
(22) Date de dépôt: 11.06.2012
(51) Int. Cl.: G01L 7/08, G01L 19/12, A61M 16/04, A61B 5/03

(54) **INDICATEUR DE PRESSION**
DRUCKANZEIGER
PRESSURE INDICATOR

(30) Priorité: 11.06.2011 FR 1101808
(43) Date de publication de la demande: 16.04.2014
(73) Titulaire: Ambu A/S, 2750 Ballerup (DK)
(72) Inventeur: DHONNEUR, Gilles, 94420 Le Plessis Trevise (FR); LUCCHINA, Pascal, 33200 Bordeau (FR); CHECCARONI, Stéphane, 06000 Nice (FR)
(74) Mandataire: Hautier, Nicolas
(86) Numéro de dépôt international: PCT/EP2012/061032
(87) Numéro de publication internationale: WO 2012/171881

(56) Documents cités:
- EP-A1- 0 228 068
- DE-C1- 3 832 252
- DE-C1- 4 116 709
- FR-A- 1 566 635
- FR-A1- 2 475 726
- GB-A- 1 464 271
- GB-A- 2 353 864
- US-A- 3 241 514
- US-A- 3 675 722
- US-A- 3 703 879
- US-A- 5 255 670
- US-A1- 2006 076 021

## Description

Le domaine technique de l'invention est celui des indicateurs de pression.

Il est connu, afin de mesurer une pression dans un fluide de faire agir ledit fluide sous pression contre une membrane. La déformation de ladite membrane est ensuite exploitée pour être traitée et affichée afin de produire une mesure indicative de ladite pression. Un tel manomètre comprend une membrane déformable sous l'effet du fluide sous pression. Afin d'obtenir une mesure la plus linéaire possible la membrane déformable d'un manomètre présente toujours une raideur élevée. Ceci conduit, pour une plage de pression donnée, pour lequel le manomètre est conçu, à des déformations très faibles de ladite membrane. Ces déformations très faibles ne sont pas visibles directement par un oeil humain et nécessitent systématiquement un dispositif de démultiplication ou d'amplification appliqué à la mesure de la déformation afin de produire une mesure de pression visible. Ceci conduit à des dispositifs complexes et le plus souvent coûteux.

Il existe par ailleurs des dispositifs comprenant un élément déformable qui se déforme sous l'effet de la présence ou de l'absence d'une pression. Lorsque la déformation de l'élément déformable atteint un seuil, l'utilisateur peut visualiser que ce seuil a été atteint.

Ces solutions sont par exemple décrites dans les documents suivants : EP 0 228 068, DE 38 32 252, FR 1 566 635, US 3 241 514, GB 1 464 271, US5255670.

Ces solutions présentent notamment comme inconvénient de permettre uniquement d'identifier a posteriori la présence ou l'absence d'une pression : pression dans un pneu de véhicule inférieure à un seuil prédéterminé (EP 0 228 068, DE 38 32 252), présence ou absence de pression au-delà d'un seuil dans une canalisation (FR 1 566 635, US 3 241 514), identification qu'une bouteille de gaz est vide (GB 1 464 271). Ces solutions ne délivrent qu'une information binaire : seuil atteint ou non. Ces solutions, tout comme celle du document US5255670 ne permettent pas d'avoir une lecture comparative et par anticipation de la pression actuelle par rapport à un seuil de pression non encore atteint. Elles ne permettent ainsi pas de prévoir à quel moment un seuil sera atteint ou de prendre par anticipation des actions permettant d'éviter qu'un seuil donné ne soit atteint.

Par ailleurs, plusieurs de ces solutions sont complexes, tant dans leur système de lecture que dans la structure de l'élément déformable.

L'invention a pour but de réaliser un indicateur de pression, de dimensions réduites, simple et peu coûteux, afin par exemple de pouvoir être à usage unique.

L'invention concerne un indicateur de pression selon la revendication 1 et comprenant un socle, une membrane déformable fixée au socle de manière étanche aux fluides selon un contour fermé, et une cloche fixée au socle, délimitant un volume creux recouvrant la membrane déformable du côté opposé au socle, en englobant au moins le contour fermé, au moins une embouchure d'entrée d'un fluide dont on veut mesurer la pression dans une plage de pression, où la membrane déformable est telle que son expansion pour ladite plage de pression soit suffisante pour être détectable à l'oeil nu et permettre un affichage direct indicatif de la pression, en ce que le socle est percé d'au moins un premier trou dont une première extrémité débouche entre la membrane déformable et le socle dans le contour fermé, en ce que la cloche est percée d'au moins un second trou dont une première extrémité débouche dans ledit volume creux, en ce que l'autre extrémité du premier trou est reliée à l'embouchure d'entrée, respectivement à l'air libre, en ce que l'autre extrémité du second trou est reliée à l'air libre, respectivement à l'embouchure d'entrée, afin qu'une pression, respectivement dépression, de fluide à l'embouchure d'entrée provoque une expansion de la membrane déformable dans le volume creux délimité par la cloche.

Selon un autre mode de réalisation, l'indicateur de pression selon l'invention comprend un socle, une membrane déformable fixée au socle de manière étanche aux fluides selon un contour fermé, et un corps solidaire du socle, délimitant un volume creux recouvrant la membrane déformable du côté opposé au socle, en englobant au moins le contour fermé, au moins une embouchure d'entrée d'un fluide dont on veut mesurer la pression dans une plage de pression. La membrane déformable est telle que son expansion (E) pour ladite plage de pression soit suffisante pour être détectable à l'oeil nu et permettre un affichage direct indicatif de la pression. Le socle est percé d'au moins un premier trou dont une première extrémité débouche entre la membrane déformable et le socle dans le contour fermé. Le corps est percée d'au moins un deuxième trou dont une première extrémité débouche dans ledit volume creux, et l'autre extrémité du premier trou est reliée à l'embouchure d'entrée, respectivement à l'air libre, l'autre extrémité du deuxième trou étant reliée à l'air libre, respectivement à l'embouchure d'entrée, afin qu'une pression, respectivement dépression, de fluide à l'embouchure d'entrée provoque une expansion de la membrane déformable dans le volume creux délimité par le corps. L'indicateur comprend au moins deux repères de pression chacun en regard d'une position d'expansion de la membrane déformable, permettant de déterminer en fonction de ladite position d'expansion de la membrane au moins trois plage de valeurs de pression.

Ainsi, chaque repère correspond à une pression prédéterminée. L'indicateur est configuré de sorte que lorsque l'expansion de la membrane atteint un repère, la pression d'une chambre disposée d'un côté de la membrane est égale à cette pression prédéterminée. Ainsi, il peut en être déduit que : lorsque la membrane n'a pas atteint le premier repère, la pression à mesurer n'a pas atteint le premier seuil de pression ; lorsque la membrane a atteint le premier repère mais n'a pas atteint le deuxième repère, la pression à mesurer est comprise entre les deux seuils de pression définis par les premier et deuxième repères; lorsque la membrane a atteint le deuxième repère, la pression est supérieure ou égale au seuil de pression défini par le deuxième repère.

Facultativement, l'invention peut en outre présenter au moins l'une quelconque des caractéristiques suivantes :
De préférence, l'indicateur comprend trois ou quatre repères.

De préférence, le corps forme une cloche. De préférence, la cloche est formée de une ou plusieurs pièces. Au moins l'une des pièces de la cloche est transparente ou translucide.

Selon l'invention le corps est solidaire du socle.

De préférence, la membrane est élastique.

De préférence, l'indicateur comprend une unique membrane déformable, la membrane étant élastique.

De préférence, la membrane est monolithique. Ainsi, elle n'est formée que d'une seule couche d'un matériau élastomère. Elle présente une structure homogène.

Selon l'invention, l'indicateur est configuré de sorte que la membrane, au cours de son expansion, prenne appui contre une paroi du corps. De préférence, l'indicateur est configuré de sorte que la membrane s'étale progressivement sur la paroi à mesure que la pression augmente. Selon l'invention, l'indicateur est configuré de sorte que lorsque la pression atteint une pression donnée, l'expansion de la membrane provoque une mise en contact de la membrane avec la paroi, puis lorsque la pression augmente, l'expansion de la membrane entraîne l'étalement de la membrane sur la paroi. De préférence, l'indicateur est configuré de sorte que l'étalement de la membrane sur la paroi est fonction de la pression à mesurer. De manière optionnelle mais préférée, la paroi s'étend dans un plan sensiblement parallèle à un plan dans lequel s'étend la membrane lorsqu'elle est au repos. La membrane encaisse le différentiel de pression jusqu'à entrer au contact de la paroi de la cloche. Le différentiel de pression est alors encaissé par la cloche.

De préférence, les repères de pression correspondent chacun à un étalement de la membrane sur la paroi. Ainsi, l'utilisateur visualise un étalement progressif de la membrane sur la paroi. L'étalement de la membrane sur la paroi du corps reflète la pression ou la dépression à observer. De préférence, l'étalement de la membrane sur la paroi du corps est proportionnel à la pression ou la dépression à observer.

De préférence la paroi est le fond d'un corps ou d'une cloche formant un volume. Elle peut également être un couvercle formant le corps ou formant partiellement le corps.
De manière optionnelle et préférée la paroi est plane.
Selon un mode de réalisation alternatif la paroi est en forme de dôme.

De préférence, la paroi est configurée de sorte à empêcher un utilisateur de visualiser la membrane lorsque la membrane n'est pas au contact de la paroi et de sorte à permettre à un utilisateur de visualiser une portion au moins de la membrane lorsque la membrane est au contact de la paroi. De préférence, l'indicateur est configuré de manière à permettre de visualiser uniquement la portion de la membrane qui est au contact de la paroi. De préférence, l'indicateur est conformé de manière à permettre de visualiser toute la portion de la membrane qui est au contact de la paroi.

De préférence, la paroi est translucide. De préférence, la membrane est opaque.

De préférence, l'indicateur comprend au moins deux et de préférence au moins trois repères disposés de sorte à identifier au moins trois positions d'étalement de la membrane, et dans lequel la membrane s'étale sous forme de disque sur la paroi.

De préférence, les repères sont disposés de sorte à visualiser l'étalement de la membrane sur une unique paroi.

Selon une autre caractéristique de l'invention, l'expansion de la membrane déformable pour la plage de pression est au moins égale à 1 mm.

Selon une autre caractéristique de l'invention, la membrane déformable est réalisée en matériau thermoplastique élastomère type TPS-SEBS ou type SBS.

La plage de pression est comprise entre 0-150 cm/H20 et où la membrane déformable présente une dureté shore A de 0 et une épaisseur comprise entre 0.3 et 0,8 mm. Selon un mode de réalisation préféré, la plage de pression est 0-120 cm/H20 et où la membrane déformable présente une dureté shore A de 0 et une épaisseur de 0,7 mm. Selon un autre mode de réalisation, la plage de pression est 0-100 cm/H₂0 et la membrane déformable présente une dureté shore A de 0 et une épaisseur de 0,5 mm.

Selon une autre caractéristique de l'invention, la membrane déformable comprend, au niveau du contour fermé, au moins un bourrelet, respectivement une gorge, coopérant avec une gorge, respectivement un bourrelet, pratiqué dans le socle.

Selon une autre caractéristique de l'invention, la membrane déformable comprend, au niveau du contour fermé, une alternance de bourrelets et de gorges, coopérant avec une alternance respectivement correspondante de gorges et de bourrelets pratiquée dans le socle.

Selon une autre caractéristique de l'invention, le matériau de la membrane déformable comprend un composé piezochrome, ou un composé tribochrome, ou un composé thermochrome.

Selon une autre caractéristique de l'invention, le matériau de la paroi, comprend un composé piezochrome, ou un composé tribochrome, ou un composé thermochrome.

Selon une autre caractéristique de l'invention, la cloche est au moins partiellement transparente afin de laisser voir l'expansion de la membrane déformable depuis l'extérieur.

Selon l'invention, la cloche comprend au moins un repère en regard d'une position d'expansion de la membrane déformable, indicatif de la pression de fluide correspondant à ladite position d'expansion.

Selon une autre caractéristique de l'invention, la cloche comprend encore au moins une lentille déformante, afin de modifier la vision de la membrane déformable en expansion.

Selon une autre caractéristique de l'invention, la lentille déformante est disposée avec son axe optique perpendiculaire à l'axe principal d'expansion.

Selon une autre caractéristique de l'invention, la lentille déformante est disposée avec son axe optique parallèle à l'axe principal d'expansion.

Selon une autre caractéristique de l'invention, la cloche comprend une extrémité proximale fixée au socle, une extrémité distale et un corps s'étendant entre les extrémités proximale et distale. Selon une option la lentille déformante est située sur le corps. De manière alternative ou cumulée avec cette option, la lentille déformante est située sur l'extrémité distale.

Selon une autre caractéristique de l'invention, la cloche est telle qu'elle appuie la membrane déformable contre le socle au niveau du contour fermé.

L'invention concerne encore une utilisation d'un tel indicateur de pression pour surveiller une pression dans un coussinet gonflable d'appareil médical, tel le coussinet d'un masque laryngé.

L'invention concerne également un dispositif médical comprenant un indicateur de pression selon l'invention et un coussinet gonflable associé à l'indicateur de manière à surveiller la pression du coussinet.

Selon un mode de réalisation avantageux, le dispositif forme un masque laryngé.

L'invention concerne également un dispositif médical pour péridurale comprenant un indicateur de pression selon l'invention et dans lequel l'un parmi le premier et le deuxième trou est en communication fluidique avec un orifice distal d'une aiguille de péridurale. Avantageusement, le dispositif médical comprend l'aiguille pour péridurale.

D'autres caractéristiques, détails et avantages de l'invention ressortiront plus clairement de la description détaillée donnée ci-après à titre indicatif en relation avec des dessins sur lesquels :
- la figure 1 montre un schéma de principe de l'invention,
- la figure 2 illustre un mode de réalisation d'une membrane déformable en vue de dessus,
- la figure 3 illustre un mode de réalisation d'une membrane déformable en vue de face,
- la figure 4 illustre un mode de réalisation d'un indicateur de pression non démontable, en vue de face,
- la figure 5 illustre un mode de réalisation d'un indicateur de pression démontable, en vue de face,
- la figure 6 illustre différents modes de comparaison d'une membrane en expansion avec un repère,
- la figure 7 illustre un mode de réalisation d'une lentille réductrice,
- la figure 8 illustre un mode de réalisation d'une lentille grossissante,
- la figure 9 illustre une utilisation.
- les figures 10 à 12 illustrent un mode de réalisation d'un indicateur de pression selon l'invention, vu en coupe.
- la figure 13 est une vue du dessus d'un indicateur de pression selon le mode de réalisation illustré en figures 10,
- les figures 14 à 17 sont des vues identiques à celle de la figure 13 et illustrent différents affichages visibles par l'utilisateur lorsque la membrane est soumise à des pressions croissantes.

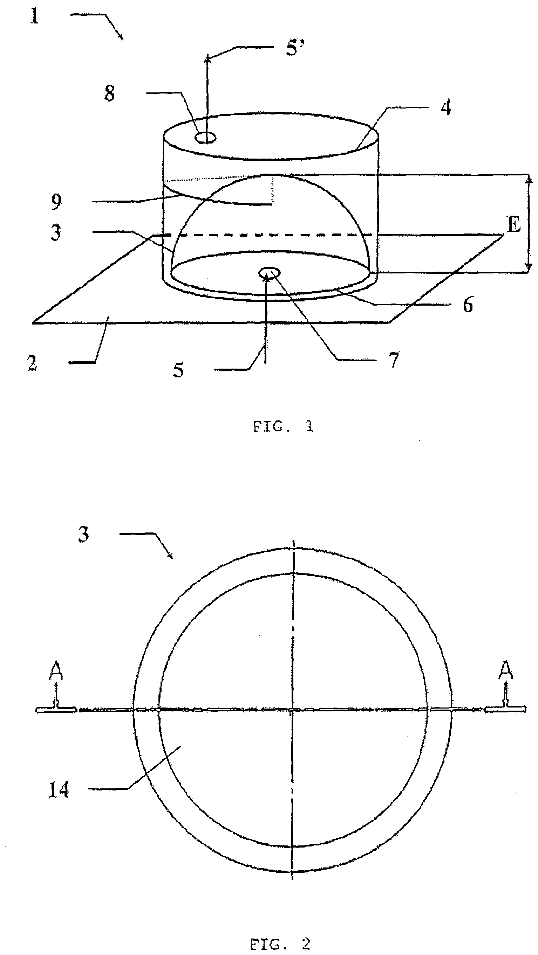

La figure 1 présente un schéma de principe de l'invention. Un indicateur de pression 1 comprend essentiellement un socle 2, une membrane déformable 3 et une cloche 4 également désignée corps. La membrane déformable 3 présente une conformation sensiblement surfacique et est fixée au socle 2 de manière étanche aux fluides selon un contour fermé 6. La surface de la membrane déformable 3 est continue dans tout l'intérieur dudit contour fermé 6.

L'indicateur de pression 1 comprend au moins une embouchure d'entrée 5, 5'. Cette embouchure d'entrée 5, 5' permet la connexion fluidique avec un circuit de fluide et ainsi l'entrée d'un fluide dont on veut mesurer la pression. Le socle 2 est percé d'au moins un premier trou 7. Une première extrémité dudit premier trou 7 débouche entre la membrane déformable 3 et le socle 2 dans le contour fermé 6. La cloche 4 est percée d'au moins un second trou 8. Une première extrémité dudit second trou 8 débouche dans ledit volume creux.

L'indicateur de pression peut être configuré selon deux configurations. Selon une première configuration, adaptée à mesurer une pression (positive), l'autre extrémité du premier trou 7 est reliée à l'embouchure d'entrée 5. Ainsi, l'embouchure d'entrée 5 est fluidiquement reliée à l'espace situé entre la membrane déformable 3 et le socle 2 et limité par le contour fermé 6, par le premier trou 7. Parallèlement, le volume creux délimité par la cloche 4 est relié à l'air libre par le second trou 8. De ce fait, l'application d'un fluide sous pression (positive) à l'embouchure d'entrée 5, par appui sur la membrane déformable 3, provoque une expansion E de la membrane déformable 3 dans le volume creux de la cloche 4.

Selon une seconde configuration, adaptée à mesurer une dépression (négative), l'autre extrémité du second trou 8 est reliée à l'embouchure d'entrée 5'. Ainsi l'embouchure d'entrée 5' est fluidiquement reliée au volume creux délimité par la cloche 4 par le second trou 8. Parallèlement, l'espace situé entre la membrane déformable 3 et le socle 2 et limité par le contour fermé 6, est relié à l'air libre, par le premier trou 7. De ce fait, l'application d'un fluide en dépression (négative) à l'embouchure d'entrée 5' provoque, par effet de vide dans le volume creux, une expansion E de la membrane déformable 3 dans le volume creux de la cloche 4.

L'expansion de la membrane déformable 3 a toujours lieu dans le même sens, la membrane déformable 3 venant remplir le volume creux, sous l'effet soit d'une pression positive appliquée entre le socle 2 et la membrane déformable 3, soit d'une pression négative/dépression appliquée au volume creux entre la cloche 4 et la membrane déformable 3.

Un indicateur de pression 1 selon l'invention peut être réalisé pour l'une des configurations avec une embouchure 5 côté socle 2 ou une embouchure 5' côté cloche 4. Selon un mode de réalisation avantageux, un indicateur de pression 1 peut être réalisé compatible des deux configurations, comprenant à la fois une embouchure 5 côté socle 2 et une embouchure 5' côté cloche 4. Le circuit dont on souhaite mesure la pression peut alors sélectivement être connectée à l'une ou à l'autre des deux embouchures 5, 5', selon le signe de la pression à mesurer, l'autre embouchure 5, 5', étant alors laissée déconnectée, à l'air libre.

Le socle est réalisé dans un matériau rigide. Un polycarbonate est un matériau possible.

Un indicateur de pression 1 est conçu pour une plage de pression déterminée. Une telle plage de pression est typiquement définie entre une pression au repos, correspondant à la pression ambiante (pression atmosphérique) et une pression maximale positive pour la configuration « pression » ou une dépression minimale négative pour la configuration « dépression ».

Selon une caractéristique importante de l'invention, la membrane déformable 3 est telle que son expansion E pour ladite plage de pression soit suffisante pour être détectable à l'oeil nu. Une telle caractéristique permet avantageusement un affichage direct, réalisé par la membrane déformable 3 elle-même, dont l'expansion E est indicative de la pression atteinte par le fluide et tient directement lieu d'index de mesure. Une telle caractéristique peut être obtenue en employant pour la membrane 3 un matériau, une dureté shore, une épaisseur, une forme, permettant d'obtenir une expansion importante, et au moins suffisante pour être directement visible, lorsque la membrane déformable 3 est soumise à une pression comprise dans la plage de pression.

Ceci permet avantageusement de faire l'économie des dispositifs de démultiplication et/ou d'amplification (pantographe, ressort, renvoi, électronique, etc.) et des dispositifs d'affichage (jauge, cadran, etc.) autrement utilisés pour les manomètres. Ceci contribue nettement à la simplification de l'indicateur de pression 1 et ainsi à la réduction de son coût.

L'indicateur de pression 1 comprend encore avantageusement une cloche 4. Ladite cloche 4 délimite un volume creux. Ladite cloche 4 est fixée au socle 2 de manière à inclure la membrane déformable 3 entre le socle 2 et la cloche 4. La membrane déformable 3 est disposée à proximité du socle 2, auquel elle est fixée au moins le long du contour fermé 6, une de ses faces regardant ledit socle 2. L'autre face de la membrane déformable 3, du côté opposé au socle 2 regarde vers le volume creux délimité par la cloche 4. La cloche 4 recouvre la membrane déformable 3 en englobant au moins le contour fermé 6. Le volume creux de la cloche 4 offre ainsi un volume d'accueil pour la membrane déformable 3 au cours de son expansion.

Afin de fixer les idées, une expansion de la membrane déformable 3 peut être considérée suffisante pour être détectable à l'oeil nu lorsque ladite expansion est au moins égale à 1 mm pour la plage de pression. La plage de pression s'étend d'une valeur de pression minimale (par exemple la pression atmosphérique) à une valeur de pression maximale en valeur absolue. L'expansion maximale est typiquement obtenue pour la valeur de pression maximale en valeur absolue. Une telle expansion maximale est avantageusement au moins égale à 1mm. Cette valeur relativement faible permet une détection visible par l'oeil humain. Cette valeur est cependant nettement plus élevée que les valeurs submillimétriques de déformation des membranes rigides des manomètres de l'art antérieur.

Cette valeur reste une valeur minimale. Afin d'améliorer la lisibilité de l'indicateur de pression, l'expansion maximale de la membrane déformable 3 pour une plage de pression/valeur de pression maximale donnée est avantageusement plus importante. Une valeur d'expansion maximale de l'ordre de 10 mm pour une plage de pression permet une lecture plus facile de l'indicateur de pression 1. Une valeur d'expansion maximale de l'ordre de 20 mm est encore plus confortable. Plus la valeur d'expansion maximale est grande en valeur absolue, plus la lecture de l'indicateur de pression 1 est aisée. Une plus grande expansion maximale permet ainsi d'augmenter le nombre de niveaux repères 9 différents et ainsi la résolution de l'indicateur de pression 1. A contrario, la valeur d'expansion maximale détermine l'encombrement selon au moins une direction de l'indicateur de pression 1. L'encombrement autorisé limite ainsi la valeur d'expansion maximale.

Il est à noter que le principe même d'une membrane déformable à forte expansion relative pour une plage de pression donnée ne permet que difficilement d'obtenir une expansion E linéaire en fonction de la pression, principalement du fait des matériaux utilisés qui présentent des comportements non linéaire en expansion. Ceci destine plus particulièrement l'indicateur de pression 1 à des utilisations où l'on souhaite comparer/surveiller une pression relativement à un ou plusieurs seuils ou à des utilisations ne nécessitant qu'une résolution grossière, plutôt qu'a des utilisations de mesure précise proprement dite.

Le principe d'une membrane déformable à forte expansion relative pour une plage de pression donnée présente cependant une très bonne répétabilité. Cette répétabilité dans le temps garantit une même indication/expansion E pour une pression appliquée donnée. Le principe de l'indicateur de pression 1 permet ainsi d'obtenir une mesure/indication avec une très bonne précision/répétabilité.

Le comportement de la membrane déformable 3 en expansion en réponse à une pression dans une plage de pression donnée dépend principalement du matériau de réalisation de la membrane déformable 3, de son épaisseur, de sa dureté shore et de la surface contenue dans le contour fermé 6, et dans une moindre mesure de la forme du contour fermé 6, de l'éventuelle mise en forme préalable de la surface utile 14 de la membrane déformable 3 dans ledit contour fermé 6, de la répartition relative des épaisseurs. S'agissant de matériaux déformables, l'expansion est très difficile, voire impossible, à calculer ou à simuler. L'homme du métier, dans ce domaine, pour déterminer ces paramètres, a recours à une approche empirique, par essais successifs. Il détermine ainsi, pour une plage de pression/une pression maximale en valeur absolue, un matériau, une épaisseur moyenne, une dureté et une surface de contour 6. Ladite pression maximale en valeur absolue est appliquée et produit une expansion maximale qui peut être comparée à celle souhaitée. En fonction du résultat obtenu, l'homme du métier modifie le matériau, ou l'un quelconque des paramètres jusqu'à obtenir ladite expansion maximale souhaitée.

Un matériau candidat, au moins pour les moyennes et basses pressions, pour la réalisation de la membrane déformable 3 un matériau thermoplastique élastomère type TPS-SEBS ou type SBS. Un tel matériau se décline en de nombreuses duretés shore et peut être fabriqué selon des épaisseurs variables afin de permettre de réaliser des indicateurs de pression pour une gamme de plages de pression très étendue.

Selon un mode de réalisation illustratif, correspondant à des basses pressions, un indicateur de pression 1 adapté à une plage de pression de 0-100 cm/H₂0 peut avantageusement être réalisé avec une membrane déformable 3 réalisée dans un tel matériau, présentant une dureté shore A de 0 et une épaisseur de 0,5 mm. Un tel mode de réalisation permet d'obtenir une expansion maximale de 20 mm avec un contour fermé circulaire de diamètre 15 mm.

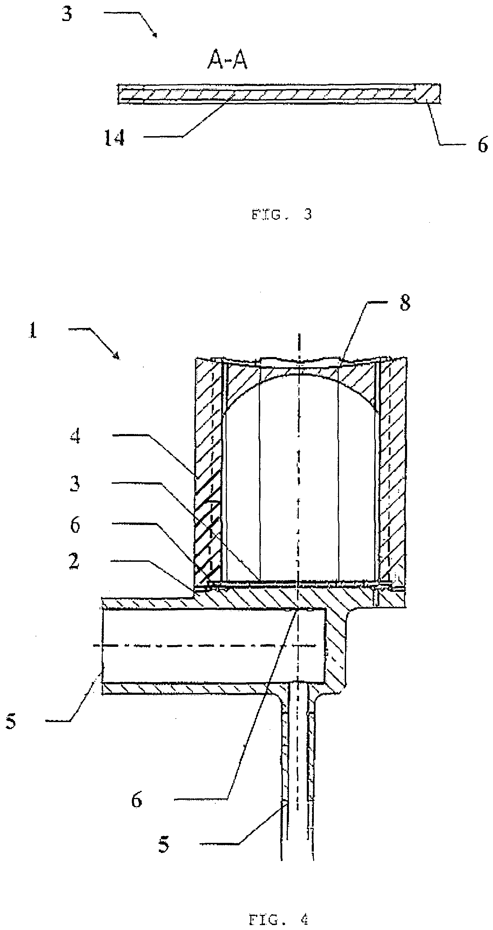

La membrane déformable 3 comprend typiquement une surface utile 14 et un contour fermé 6. La forme du contour fermé 6 peut être quelconque. Les figures 2 et 3 illustrent un mode de réalisation d'une membrane déformable 3 présentant un contour fermé 6 circulaire. La forme de la surface utile 14, limitée par ledit contour fermé 6 peut être l'objet d'une mise en forme préalable. Une forme préalable de coquille sphérique peut par exemple être employée pour faciliter l'expansion à son début. Le mode de réalisation illustré aux figures 2 et 3 présente une forme initiale plane.

Pour les matériaux élastomères, la membrane déformable 3 est avantageusement réalisée par moulage de précision, par injection thermoplastique. Ceci permet une excellente répétabilité de fabrication qui permet avantageusement de garantir une bonne répétabilité des mesures de pression d'un indicateur de pression à l'autre. Ceci permet une réalisation en grande ou très grande série.

La membrane déformable 3 est avantageusement réalisée monobloc comprenant la surface utile 14 est le contour fermé 6, avec tous ses éventuels aménagements.

La fixation de la membrane déformable 3 au socle 2 doit être réalisée de manière étanche aux fluides le long du contour fermé 6. Cette fixation peut être réalisée selon tout moyen connu de l'homme du métier tel que collage, soudage, bridage, etc.

Selon un mode de réalisation illustré par exemple à la figure 4, particulièrement avantageux, ladite fixation est réalisée par appui du contour fermé 6, sur toute sa longueur, contre le socle 2. Ceci est avantageusement facilité par un chemin, complémentaire du contour fermé 6, réalisé sur une paroi du socle 2. Dans ce cas le contour fermé 6 présente avantageusement une surépaisseur relativement à la surface utile 14 afin de permettre une compression assurant l'étanchéité aux fluides.

Selon un mode de réalisation préférentiel, toujours illustré à la figure 4, ledit appui est réalisé par la cloche 4. La cloche 4 vient se fixer au socle 2 et est conformée de manière à venir appuyer le contour fermé 6 contre le socle 2.

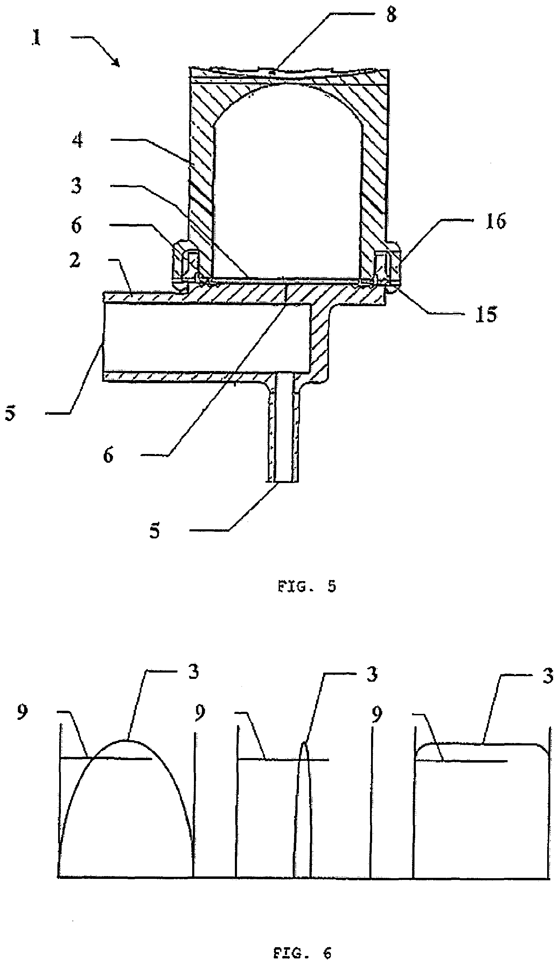

La fixation de la cloche 4 sur le socle 2 peut être réalisée de différentes manières. Le socle 2 et la cloche 4 présentent avantageusement des surfaces complémentaires pour faciliter ledit assemblage. Il est possible de réaliser un assemblage non démontable, tel qu'illustré à la figure 4. Dans l'exemple illustré le socle 2 et la cloche 4 comportent des alésages complémentaires en regard. La cloche 4 est mise en place sur le socle 2. L'assemblage peut ensuite être bloqué par tout moyen connu : collage, soudage, etc. Il est encore possible de réaliser un assemblage démontable, tel qu'illustré à la figure 5. Dans l'exemple illustré, la cloche 4 et le socle 2 présentent des moyens élastiques complémentaires d'assemblage par clippage. Le socle 2 est muni d'un ergot circulaire 15, sur lequel au moins une patte élastique 16 de la cloche 4 vient se clipper. Ainsi la cloche 4 peut être clippée sur le socle 2. Les pattes élastiques 16 peuvent être déformées de manière élastique afin de permettre un démontage.

Quel que soit le mode d'assemblage (collage, soudage, par compression/appui, etc.) de la membrane déformable 3 sur le socle 2, il convient de réaliser une étanchéité aux fluides entre ces deux pièces 2, 3 tout au long du contour fermé 6. Cette étanchéité peut être réalisée par le mode d'assemblage lui-même. Cette étanchéité peut avantageusement être renforcée en réalisant des surfaces en regard toujours complémentaires mais non nécessairement lisses. Ainsi la membrane déformable 3 comprend avantageusement, au niveau du contour fermé 6, au moins un bourrelet, coopérant avec une gorge, pratiquée dans le socle 2. De manière analogue, la membrane déformable 3 comprend avantageusement, au niveau du contour fermé 6, au moins une gorge, coopérant avec un bourrelet, pratiqué dans le socle 2.

Il est encore possible de combiner plusieurs paires bourrelet/gorge successives afin de renforcer encore l'étanchéité obtenue. Une alternance de bourrelets et de gorges est préférée. Ainsi la membrane déformable 3 comprend avantageusement, au niveau du contour fermé 6, une alternance de bourrelets et de gorges, coopérant avec une alternance respectivement correspondante de gorges et de bourrelets pratiquée dans le socle 2.

Ladite gorge et/ou bourrelet sont complémentaires et sensiblement parallèles au contour fermé 6. Ladite gorge et/ou bourrelet est avantageusement réalisé de matière avec le socle 2/la membrane déformable 3, par tout moyen de réalisation : injection, usinage, etc.

La cloche 4 a plusieurs fonctions. Il est possible de distinguer au moins une fonction de protection, une fonction de repérage, et une fonction de saturation/sauvegarde.

Une première fonction de protection consiste à protéger le volume dans lequel la membrane déformable 3 s'expanse, afin que ladite expansion ne soit pas empêchée par un obstacle. Pour cela la cloche 4 est réalisée dans un matériau rigide empêchant l'indicateur de pression 1 d'être écrasé par un élément de l'environnement.

Dans cette fonction, en configuration « pression » la cloche 4 ne doit cependant pas empêcher ladite expansion de la membrane déformable 3. Aussi afin de ne pas produire une contre pression qui s'opposerait à l'expansion, le volume creux délimité par la cloche 4 est avantageusement mis à l'air libre. Ceci est réalisé par ledit au moins un second trou 8 percé dans l'épaisseur de la cloche 4 de manière à relier le volume creux à l'air libre. Ainsi lors de l'expansion de la membrane déformable 3, l'air contenu dans le volume creux peut librement s'échapper.

De manière analogue en configuration « dépression », le volume situé entre la membrane déformable 3 et le socle 2 est avantageusement mis à l'air libre. Ceci est réalisé par ledit au moins un premier trou 7 percé dans l'épaisseur du socle 2. Ainsi lors de l'expansion de la membrane déformable 3, de l'air de l'extérieur peut librement entrer dans le volume situé entre la membrane déformable 3 et le socle 2, et autoriser ainsi l'expansion de la membrane déformable 3.

Le ou les premiers trous 7, respectivement seconds trous 8, présentent conjointement une surface suffisamment grande pour assurer la mise en pression/dépression et/ou la mise à l'air libre. Cependant, chaque premier trou 7, respectivement second trou 8, pris individuellement présente une surface suffisamment petite pour interdire une extrusion de la membrane déformable 3 à travers lui.

Cette dernière caractéristique peut être assurée, y compris pour des pressions nettement inférieures ou nettement supérieures aux pressions comprises dans la plage de pression. La cloche 4 garantit alors que la membrane déformable 3 ne peut s'expanser ni en deçà ni au delà du volume creux. Ceci assure avantageusement une protection de la membrane déformable 3, empêchant sa destruction par éclatement en cas de sous-pression ou de surpression, y compris en cas d'inversion du signe de la pression. Ceci assure une double saturation basse et haute bénéfique qui réalise une fonction de sauvegarde de l'indicateur de pression 1.

Une autre fonction de la cloche est, dans une moindre mesure, de réaliser un guidage de la membrane déformable 3 lors de son expansion. La membrane déformable 3 en l'absence de cloche 4 présente une expansion sphérique. La forme intérieure de la cloche 4, par exemple cylindrique dans les figures 4 et 5, contraint la membrane déformable 3 à une expansion cylindrique.

Une autre fonction importante de la cloche 4 est de permettre un repérage de la pression. Pour cela la cloche 4 est, au moins partiellement, transparente. Ainsi l'expansion E de la membrane déformable 3 est directement visible depuis l'extérieur de l'indicateur 1 et peut être observée par un utilisateur. La cloche 4 est par exemple réalisée en verre ou en polycarbonate.

Afin de compléter cette fonction de repérage, la cloche 4 comprend selon l'invention au moins un repère 9, en regard d'une position d'expansion de la membrane déformable 3, indicatif de la pression de fluide correspondant à ladite position de d'expansion. Il est ainsi possible de comparer une position d'expansion E de la membrane déformable 3 avec un repère 9 et d'obtenir relativement une mesure de la pression de fluide et ceci directement par rapport à un repère 9 avantageusement indicatif d'un niveau de pression seuil ou caractéristique. Ainsi en référence à la figure 1, la membrane déformable 3 est dans une position d'expansion correspondant à une expansion E la plaçant en regard du repère 9. Ce repère 9 ayant été préalablement calibré, la pression, respectivement dépression, correspondant à cette configuration est alors déterminée.

Il est ainsi possible de réaliser un témoin, comprenant un repère 9 séparant deux zones ou deux repères correspondant à deux positions d'expansion et donnant une indication tout ou rien de la pression. Il est encore possible de réaliser un indicateur 1 comprenant n repères 9 et permettant de comparer la pression de fluide à ces n repères 9. Ledit nombre n peut être quelconque est n'est limité que par la dimension longitudinale de l'indicateur de pression 1 selon l'axe principal d'expansion et par la résolution de l'oeil de l'utilisateur.

Ledit au moins un repère 9 peut être réalisé par tout moyen connu. Une gravure en creux ou en plein, à l'intérieur ou à l'extérieur de la cloche 4 est possible. Le repère 9 peut encore être imprimé ou sérigraphié. Le repère 9 peut encore être réalisé par une coloration/teinture dans la masse ou en surface de la cloche 4 ou encore une partie opaque de cette dernière.

En se référant à la figure 6, selon les dimensions relatives de la membrane déformable 3 et de la cloche 4, la déformation de la membrane déformable 3 prend une forme sensiblement de dôme sphérique. La partie utile de la membrane déformable 3, qui est comparée aux repères 9 est en forme de dôme. Cette forme rend difficile la comparaison de la position dudit dôme avec la position d'un repère 9, comme illustré au schéma de gauche de la figure 6.

Afin de remédier à cet inconvénient, la cloche 4 comprend encore au moins une lentille déformante 10, 11 afin de modifier la vision de la membrane déformable 3 en expansion. Toujours en référence à la figure 6, le schéma central illustre la membrane déformable 3 telle que vue au travers d'une lentille 10 réductrice, telle une lentille cylindrique dont l'axe du cylindre est disposé parallèle à l'axe principal d'expansion (verticale dans le plan de la figure). Une telle lentille réductrice 10 transforme une forme de membrane déformable 3 en dôme, en une forme en bâtonnet. Le dôme est alors remplacé par l'extrémité dudit bâtonnet, ce qui permet une comparaison visuelle avec le repère 9 plus aisée.

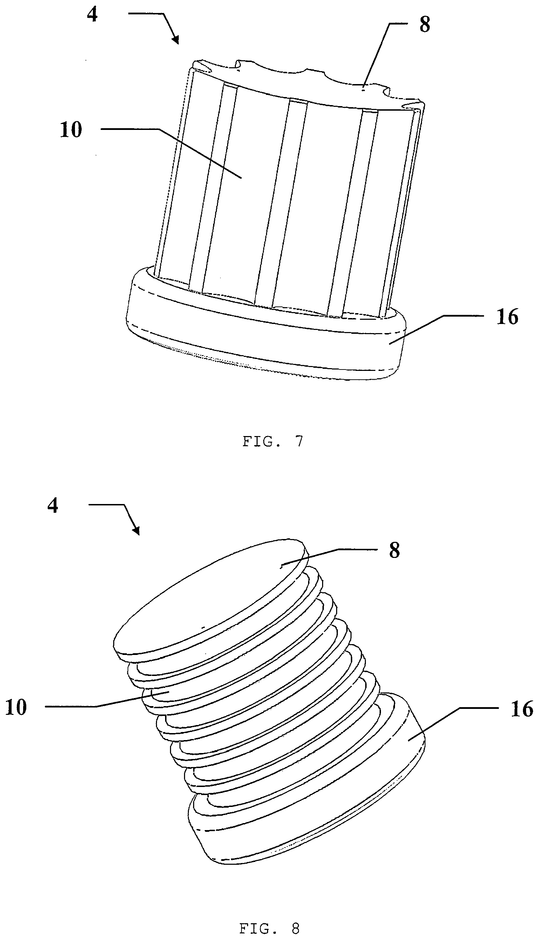

La figure 7 illustre, selon un mode de réalisation, une cloche 4 comprenant sur toute sa périphérie des lentilles réductrices 10 cylindriques dont l'axe de cylindre est disposé parallèle à l'axe principal d'expansion.

Toujours en référence à la figure 6, le schéma de droite illustre la membrane déformable 3 telle que vue au travers d'une lentille 11 grossissante, telle une lentille cylindrique dont l'axe du cylindre est disposé perpendiculaire à l'axe principal d'expansion (horizontale dans le plan de la figure). Une telle lentille grossissante 11 transforme une forme de membrane déformable 3 en dôme, en une forme en plateau. Ledit plateau permet une comparaison visuelle avec le repère 9 plus aisée.

La figure 8 illustre, selon un mode de réalisation, une cloche 4 comprenant sur toute sa hauteur des lentilles grossissantes 11 cylindriques dont l'axe de cylindre est disposé perpendiculaire à l'axe principal d'expansion.

Dans les deux modes de réalisation précédents, les lentilles 10, 11 sont disposées sur la paroi cylindrique de la cloche 4. Elles se caractérisent par un axe optique de lentille perpendiculaire, dans les deux cas, à l'axe principal d'expansion (axe de révolution de la cloche 4).

Selon un autre mode de réalisation, illustré aux figures 4, 5, 7 et 8, il est encore possible de disposer une lentille dont l'axe optique soit parallèle à l'axe principal d'expansion (axe de révolution de la cloche 4). Ceci est réalisé par exemple par une concavité à l'extrémité distale de la cloche 4, visible en partie supérieure dans le plan des figures. Une telle lentille offre un autre point de vue sur la déformation de la membrane déformable 3, qui peut être très significative au regard d'un changement de pression.

Afin de remplacer, ou de compléter, l'indication de pression obtenue par comparaison de l'expansion E de la membrane déformable 3 avec au moins un niveau indiqué par un repère 9, il est possible de rajouter un effet visible afin d'attirer encore davantage l'attention d'un utilisateur. Un tel effet peut par exemple être un effet de changement de couleur.

En intégrant dans le matériau de la membrane déformable 3, lors de sa fabrication, un composé piezochrome ou tribochrome, la membrane déformable 3, lorsque soumise à une pression, en plus de l'expansion E précédemment décrite et créant un effet spatial, va avantageusement présenter un changement de couleur, créant un effet chromique, renforçant ainsi l'effet spatial et augmentant les chances d'attirer l'attention de l'utilisateur.

Selon un autre mode de réalisation alternatif ou complémentaire, il est aussi possible d'intégrer dans le matériau de la membrane déformable 3, lors de sa fabrication, un composé thermochrome. Ceci permet de compléter la fonction d'indicateur de pression 1, par une fonction d'indication de température du fluide observé. Ceci peut s'avérer très avantageux, par exemple dans une application de surveillance médicale, où un même indicateur permet de réaliser une double surveillance de la pression et de la température.

De manière avantageuse, l'indicateur est configuré de sorte que la membrane, au cours de sa déformation, prenne appui contre une paroi du corps, puis s'étale progressivement sur cette paroi à mesure que la pression augmente. Ainsi, l'utilisateur visualise un étalement progressif de la membrane sur la paroi. L'étalement de la membrane sur la paroi du corps reflète la pression ou la dépression à observer. De préférence, l'étalement de la membrane sur la paroi du corps est proportionnel à la pression ou la dépression à observer.

De préférence, la paroi est le fond d'un corps ou d'une cloche formant un volume creux. Elle peut également être un couvercle formant le corps ou formant partiellement le corps. Elle est solidaire du socle de l'indicateur.

Selon l'invention, l'indicateur de pression comporte des repères qui permettent de visualiser le niveau d'étalement de la membrane sur la paroi. Ces repères correspondent par exemple chacun à une pression prédéfinie qui peut être indiquée sur l'indicateur lui-même ou sur un élément associé. L'utilisateur peut donc relever différents niveaux de pression en observant le repère atteint par la portion de membrane au contact de la paroi. En fonction de la disposition et du nombre de repères, l'indicateur de pression permet ainsi de mesurer une pression.

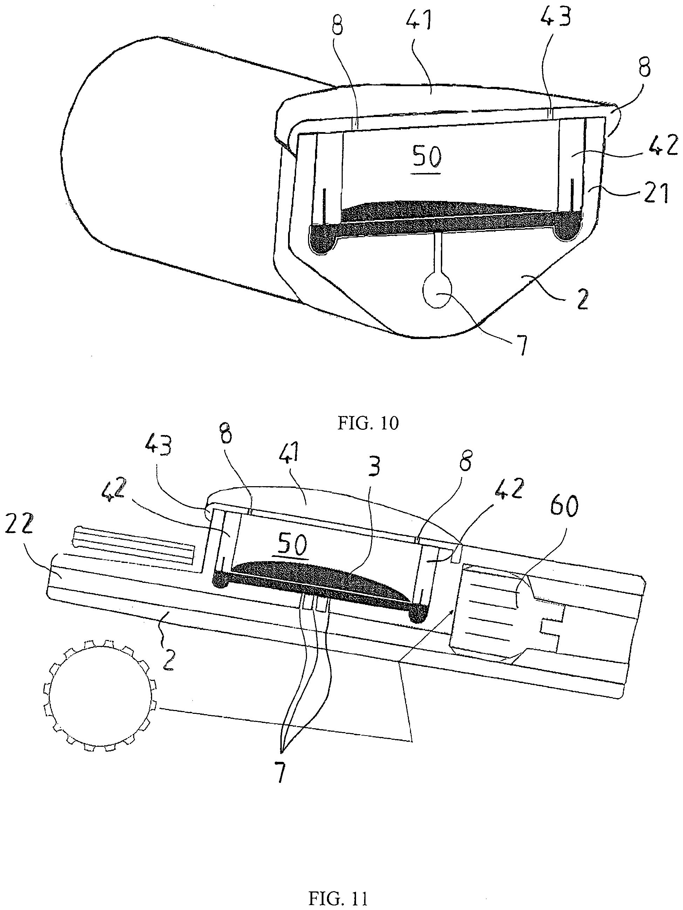

Ce mode de réalisation est particulièrement avantageux puisqu'il permet de limiter l'effort transmis par le fluide sous pression à la membrane. En effet cet effort, une fois la membrane au contact de la paroi, est transmis au moins en partie à la paroi. La membrane se déforme moins que s'il n'y avait pas de contact avec la paroi. Elle est ainsi moins sujette à la fatigue et permet d'améliorer la reproductibilité des mesures même après un grand nombre d'utilisations. Cet aspect sera expliqué plus en détail par la suite après avoir présenté un exemple de réalisation en référence aux figures 10 à 17.

De manière avantageuse, l'indicateur de pression est conformé de sorte que la membrane n'est pas visible par l'utilisateur avant de prendre contact sur la paroi. Ainsi, l'utilisateur ne peut visualiser que la portion de membrane au contact de la paroi. Il peut ainsi mieux observer la position de l'étalement ou l'évolution de l'étalement de la membrane à la surface de la paroi.

Les figures 10 à 12 font apparaître un indicateur de pression reprenant les caractéristiques mentionnées précédemment à propos des autres modes de réalisation. En particulier, ces figures font apparaître la membrane 3 enserrée entre une pièce 42 du corps et le socle 2. Dans cet exemple, le corps comprend une pièce 42, par exemple de forme cylindrique et de préférence de section circulaire, et un couvercle 41 obstruant une extrémité de la pièce 42. La face supérieure de la membrane 3, la pièce 42 et le couvercle 41 délimitent un premier volume creux 50, également désigné première chambre à l'intérieur de laquelle la membrane peut se déformer sous l'effet d'une pression. Le corps comprend au moins un trou 8 pour une mise en communication du premier volume creux 50 avec l'ambiant ou avec une chambre dont la pression doit être mesurée. Sur l'exemple illustré, le couvercle 41 présente un trou 8 en communication avec l'air libre pour maintenir le premier volume creux 50 à pression atmosphérique. De préférence, le couvercle 41 comprend plusieurs trous 8. La liaison entre la membrane 3 et la pièce 42 est étanche.

Le socle 2 présente au moins un trou 7, de préférence plusieurs comme illustré en figure 11, en communication fluidique avec un conduit 22, lui-même en communication fluidique avec la chambre dont la pression est à mesurer. Avantageusement, en position de repos la membrane 3 repose sur le socle 2. Cela permet de supprimer ou de réduire un espace entre le socle 2 et la membrane, permettant ainsi de réduire l'encombrement de l'indicateur de pression. Eventuellement, cela permet aussi de ne pas imposer à la membrane 3 une sollicitation permanente sous l'effet de sa masse.

Avantageusement, le conduit 22 est traversant et une valve 60 peut être prévue pour obstruer le conduit 22 en aval des trous 7.

Sur l'exemple illustré, le socle 2 présente un logement défini par des parois 21. Le logement est configuré pour accueillir la pièce 42 du corps. De préférence, le couvercle 41 se fixe sur le logement. La robustesse et la compacité de l'indicateur en sont améliorées. Cette fixation comprend par exemple un clippage, un collage et ou un vissage. Des rebords 43 prévus sur le couvercle 41 contribuent à cette fixation.

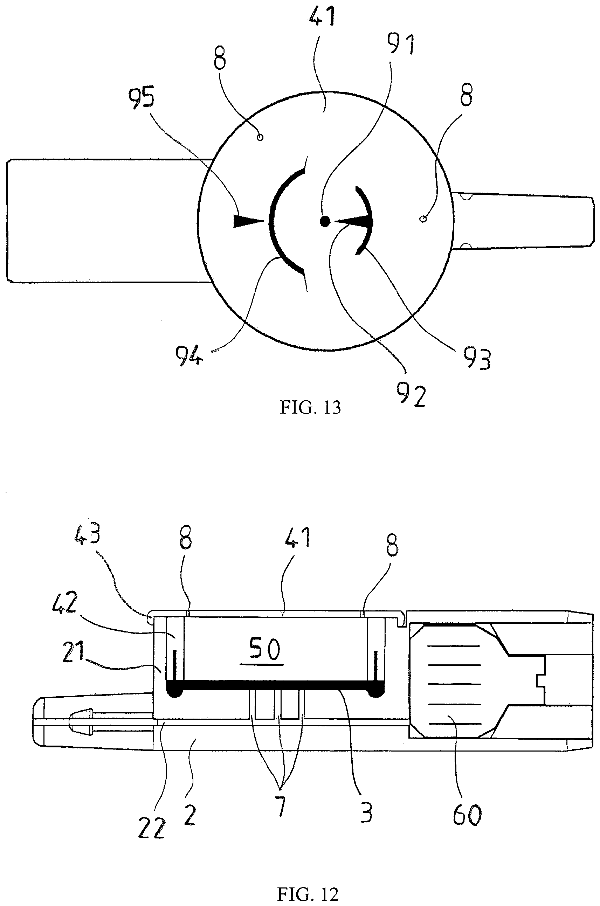

Comme illustré sur les figures 13 à 17, le corps porte des repères 91, 92, 93, 94, 95 tous visibles en figure 13. Comme indiqué précédemment, ces repères sont par exemple sérigraphiés, collés ou gravés sur le corps.

Ainsi, l'indicateur de pression selon l'invention est basé sur une visualisation directe de la déformation d'une membrane élastomère afin d'indiquer à un utilisateur des niveaux de pression/dépression dans un circuit sans avoir à recourir à un mécanisme complexe d'amplification d'une déformation de faible amplitude. L'indicateur de pression selon l'invention nécessite alors une déformation importante de la membrane pour être perçue par l'oeil humain et par voie de conséquence une grande souplesse de la membrane surtout lorsqu'il s'agit de mesurer des basses pressions. Or, tout matériau souple type élastomère ou autres, soumis à des déformations constantes étirements/contractions successifs et continus fait l'objet d'une fatigue plus ou moins importante d'où des mesures de pressions relativement aléatoires, imprécises et non récurrentes dans le temps. Pour sécuriser l'information des niveaux de pression transmis par l'indicateur de pression selon l'invention, c'est-à-dire pour assurer une linéarité et une récurrence des indications correspondant aux divers niveaux de valeurs de pressions, l'invention prévoit de limiter volontairement la déformation de la membrane souple, mais, sans augmenter la raideur de la membrane et sans ajout de dispositifs de démultiplication et d'amplification du mouvement comme dans les solutions de l'art antérieur.

A cet effet, l'invention prévoit une limitation extrême de la hauteur d'extension de la membrane 3 soumise à la pression. Dans ce mode de réalisation, la déformation maximale de la membrane 3 est contrôlée et conduite à se cantonner dans une plage d'expansion se situant avant le début de la fatigue du matériau souple et ce, indépendamment et quelle que soit la valeur de la pression appliquée.

Ce mode de fabrication particulier permet ainsi d'obtenir l'assurance d'une récurrence optimale de la forme structurelle de la déformation que revêt la membrane 3 lorsqu'elle est soumise séquentiellement à divers niveaux de pression d'un fluide et la perception visuelle claire à l'oeil nu, sans ajout d'appareils démultiplicateurs ou amplificateurs.

Avec cette structure d'indicateur selon l'invention, le matériau élastomère de la membrane est alors capable de supporter un nombre très grand, voire quasi illimité, de séquences de gonflages et dégonflages, dans toute plage de durée, sans modification dans le temps de la forme structurelle de sa déformation tout en permettant à l'utilisateur de visualiser sur le couvercle de la cloche une grande amplitude de déformation selon les niveaux de pressions appliqués.

Ce mode de fabrication représente une avancée majeure de la technique car il résout le problème non résolu à ce jour des matériaux élastiques en ce qu'ils possèdent une résistance limitée à la fatigue lorsqu'ils sont soumis à des séquences d'étirement/contraction constants. Cette caractéristique de l'invention rend en effet la membrane élastomère quasiment insensible à toute fatigue du matériau permettant ainsi d'obtenir une cinématique constante de la forme structurelle des déformations quels que soient les niveaux de durée et de pression auxquels elle est soumise, tout en permettant une large visualisation de déformations selon les niveaux de pression appliqués.

Grâce à cette nouvelle caractéristique l'invention est plus sûre et l'indication de pression plus fiable. Pour ce faire la membrane 3 élastomère de couleur préférentiellement opaque, typiquement noir, est alors placée à faible distance du couvercle 41 du corps ou plus généralement de la cloche.

De manière particulièrement avantageuse, la paroi contre laquelle vient s'étaler la membrane en expansion empêche la visualisation de la membrane 3 avant que ce contact ne s'établisse et permet de visualiser la membrane 3 lorsque le contact est établi. Ainsi le couvercle 41 ou le plafond de la cloche est fabriqué dans une matière rigide et non transparente. De préférence, la paroi (couvercle 41 ou plafond de la cloche) contre laquelle la membrane est destinée à entrée au contact et à s'étaler est translucide de sorte à empêcher l'utilisateur de visualiser les parties de membrane qui ne sont pas à son contact. Cette paroi est préférentiellement de couleur blanchâtre pour obtenir un contraste optimal avec la membrane de couleur noire.

La membrane 3 au repos (non gonflée), donc sans contact avec la surface du couvercle est alors quasi invisible depuis l'extérieur du dispositif par l'oeil de l'utilisateur car dissimulée par la translucidité du matériau de la paroi 41 qui fait écran à la transmission de la lumière vers l'intérieur du volume creux 50 et donc, voile la visualisation de la membrane 3 alors non soumise à une pression. Sous l'action de la pression du fluide, la membrane vient alors immédiatement se mettre en contact sans effort avec la sous face interne de la paroi translucide. De manière naturelle, le premier contact entre la paroi et la membrane s'effectue toujours en un même point, typiquement au centre du couvercle ou du plafond de la cloche de forme circulaire. Lorsque ce contact s'établie, l'oeil de l'utilisateur voit alors apparaître un point de couleur gris foncé ou noir en ce point de la paroi translucide du plafond de la cloche.

Ensuite, plus la pression du fluide augmente, plus la membrane s'étale sur la face interne de la paroi. L'indicateur est de préférence configuré de manière à ce que toute la portion de membrane au contact de la paroi soit visible par l'utilisateur et à ce que la portion de membrane qui n'est pas au contact de la paroi ne soit pas visible ou ne soit pas nettement visible. L'étalement de la membrane s'effectue par exemple de manière circulaire sur la paroi interne du couvercle passant alors sans contrainte, par exemple de 0 à 15 mm de diamètre pour des pressions de 0 à 120 cm/H2O. Dans cette configuration particulière et innovante, le matériau souple de la membrane n'est alors soumis à aucune fatigue puisque l'effort résultant de la pression est directement transféré au matériau rigide de la paroi du corps.

L'oeil de l'utilisateur voit alors s'étendre, le point initial central de couleur noir, depuis le centre de la surface externe blanche translucide du couvercle 41 en direction des bords extrêmes délimités par la circonférence de ce dernier. En pression maximale la surface fonctionnelle du couvercle devient alors totalement noire.

La surface du couvercle peut être alors dotée de graduation permettant de transmettre visuellement à l'utilisateur l'indication de la valeur réelle de la pression du fluide.

Les figures 13 à 17 illustrent différents niveaux de pression qui peuvent être mesurés à grâce à l'indicateur selon l'invention.

En figure 13, la paroi 41 laisse apparaître tous les repères 91-95. La membrane n'apparaît pas, ce qui signifie que la pression à mesurer est inférieure à la pression correspondant au repère 91.

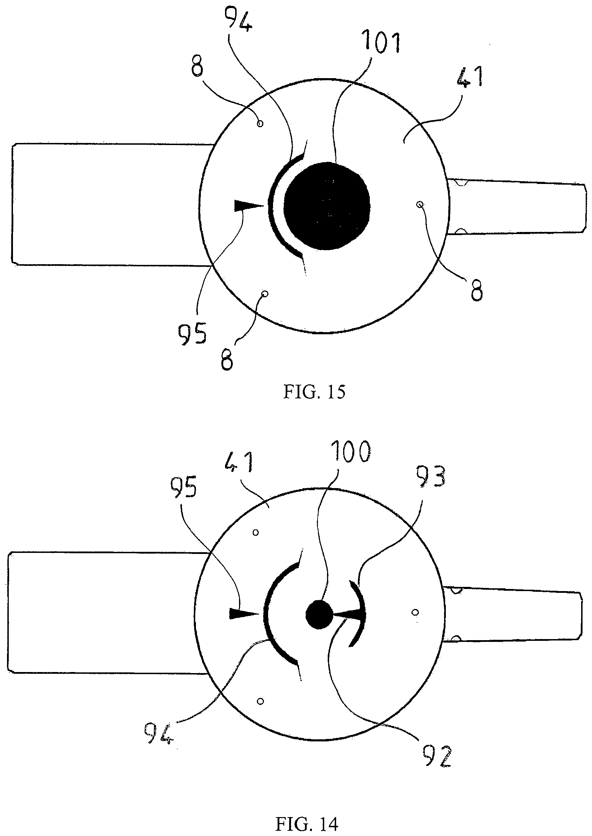

En figure 14, une tache 100 apparaît. Cette visualisation est permise par la mise en contact de la membrane 3 avec la face interne de la paroi 41. La surface de cette tache correspond à surface de la portion de membrane 3 qui est au contact de la face interne de la paroi 41. Le repère 92 affleure la tache 100 et permet de lire que la pression à mesurer est égale à la pression associée au repère 92.

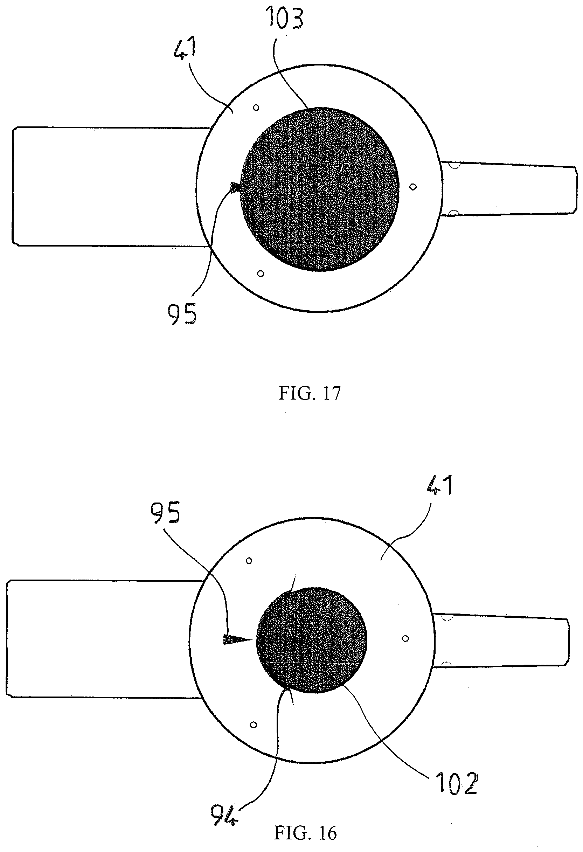

En figure 15, la tache 101 s'est étalée et affleure le repère 93, signifiant que la pression dans la chambre est égale à la pression associée au repère 93. Au fur et à mesure que la pression à mesurer s'accroît, la membrane s'étire et s'étale sur la paroi 41. La surface de l'étalement grandit et atteint le repère 94 comme illustré en figure 16, signifiant que la pression à mesurer est égale à la pression associée au repère 94.

Sur la figure 17, la pression à mesurer a atteint la pression associée au repère 95.

La membrane peut ainsi s'étendre jusqu'à être plaquée contre toutes les parois délimitant le volume creux 50. Comme ce volume creux 50 est délimité par des parois rigides, ce volume est constant quelle que soit la pression à mesurer. Ainsi, l'invention permet de limiter l'expansion de la membrane à un volume égale à celui de la chambre 50 quelle que soit la pression à mesurer. En choisissant une membrane dont la limite de déformation plastique est supérieure au volume de la chambre 50 on est assuré que la membrane conservera son élastique. Ainsi, même si la pression à mesurer dépasse une pression préconisée d'utilisation, la membrane n'en sera pas affectée et ses mesures seront toujours aussi fiables.

Pour ce mode de réalisation, on pourra par exemple prévoir de disposer la membrane à moins de 5 millimètres de la paroi pour identifier des pressions comprises entre 0 à 120 cm/H2O. De préférence, la membrane déformable présente une dureté shore A de 0 et une épaisseur de 0,7 mm. Les disques visibles à la surface de la paroi 41 présenteront un diamètre compris entre 0 et 15 millimètres.

Toutes les caractéristiques mentionnées, précédemment notamment concernant les matériaux pour former la membrane, s'appliquent également à ce mode de réalisation.

L'invention s'étend également aux parois non translucides et qui permettent de masquer la membrane en l'absence de contact entre cette dernière et la paroi et qui permettent à un utilisateur de visualiser la membrane lorsque cette dernière est au contact de la paroi. De préférence, la paroi permet de visualiser uniquement la portion de membrane au contact de la paroi.

Ainsi, en plus des paroi translucide, l'invention s'étend aux parois qui empêchent la visualisation de la membrane par réflexion de la lumière, réflexion qui est amoindrie ou supprimer lorsque la membrane est au contact d'une face de la paroi.

L'invention s'étend également aux indicateurs dont la paroi entrant au contact de la membrane n'est pas une paroi sensiblement parallèle à la membrane dans sa position de repos.

La réalisation d'un tel indicateur de pression 1 nécessite peu de pièces, typiquement trois. Ces pièces sont aisément réalisables en grande série et permettent d'obtenir un produit peu cher. Il est ainsi possible de réaliser un indicateur de pression 1 à usage unique, ce qui est particulièrement appréciable pour les applications médicales.

De plus le principe de l'invention par expansion d'une membrane déformable 3 permet de réaliser un indicateur de pression dans un encombrement très réduit.

Le principe de l'invention, où la membrane présente une masse très faible relativement à la pression mesurée, permet de négliger les effets de la gravitation. Il en résulte qu'un indicateur de pression 1 selon l'invention est avantageusement insensible à l'orientation et peut être mis en oeuvre dans toutes les positions/orientations.

Un tel indicateur de pression 1 peut être utilisé dans de nombreuses applications. Une application particulièrement avantageuse est dans le domaine médical pour toute application de surveillance de pression/dépression dans des plages de pression basse. Ainsi dans le cadre d'appareil d'intubation à usage respiratoire, il est connu d'utiliser des coussinets gonflables.

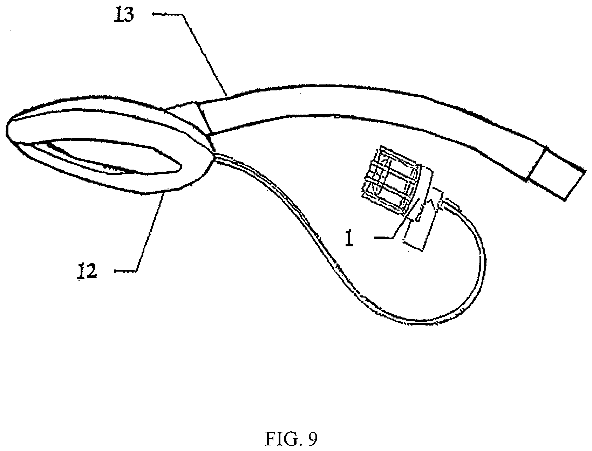

La figure 9 illustre un exemple où un tel appareil est un masque laryngé 13 venant recouvrir l'entrée du larynx après mise en place dans la cavité buccale d'un patient. Un tel appareil assure son étanchéité au moyen d'un coussinet gonflable 12. Il convient que le coussinet 12 soit suffisamment gonflé pour que cette étanchéité soit assurée. Cependant, les zones de la cavité buccale sur lesquelles appuie ledit coussinet 12 sont critiques en ce qu'elle comporte des artères cérébrales. Une pression trop importante sur ces artères peut entraîner des dommages cérébraux irréversibles et doit impérativement être évitée.

Un indicateur de pression 1 selon l'invention adapté à une plage de pression de 0-100 cm/H₂O est avantageusement connecté en piquage sur le coussinet 12 pour surveiller la pression dans le coussinet 12, et donc la pression d'appui sur la cavité buccale du patient, au cours de son gonflage. Un tel indicateur de pression 1 est avantageusement équipé de au moins deux repères 9. Un premier repère correspond à une pression minimale de gonflage du coussinet 12 et à une valeur de 40cm/ H₂O. Un second repère correspond à une pression limite de dommage physiologique à ne pas dépasser et à une valeur de 80cm/ H₂O. Ainsi, au moyen de l'invention, un opérateur peut efficacement surveiller la mise en place et le gonflage du coussinet 12, en toute sécurité pour le patient.

L'invention s'étend à de nombreux dispositif dans lesquels l'indicateur selon l'invention peut s'intégrer. Ainsi par exemple, l'indicateur selon l'invention peut aussi être intégré dans un dispositif pour pratiquer une péridurale. L'indicateur de pression est alors utilisé en association avec une aiguille de péridurale. Plus précisément il est conçu pour confirmer la position du biseau de l'aiguille dans l'espace péridural. L'un des deux trous de l'indicateur est mis en communication fluidique avec l'orifice distal de l'aiguille d'une seringue de péridurale. La levée de la pression dans le système fermé et le dégonflage de la membrane indiquent au praticien que le biseau de l'aiguille a pénétré dans un vide ou que le système est ouvert pour une autre raison. Le praticien a ainsi une indication que le biseau de l'aiguille a pénétré dans l'espace péridural.

Cette application pour péridurale est applicable à tous les modes de réalisation précédemment décrits.

Bien qu'il soit décrit dans la présente un mode de réalisation préféré de l'invention, il doit être bien compris que l'invention n'est pas limitée à ce mode, et que des variations peuvent être apportées à l'intérieur de la portée des revendications suivantes.

## Revendications

1. Indicateur de pression comprenant un socle (2), une membrane déformable (3) fixée au socle (2) de manière étanche aux fluides selon un contour fermé (6), et un corps (4) solidaire du socle (2), délimitant un volume creux recouvrant la membrane déformable (3) du côté opposé au socle (2), en englobant au moins le contour fermé (6), au moins une embouchure d'entrée (5, 5') d'un fluide dont on veut mesurer la pression dans une plage de pression, dans lequel la membrane déformable (3) est telle que son expansion (E) pour ladite plage de pression soit suffisante pour être détectable à l'oeil nu et permettre un affichage direct indicatif de la pression, dans lequel le socle (2) est percé d'au moins un premier trou (7) dont une première extrémité débouche entre la membrane déformable (3) et le socle (2) dans le contour fermé (6), ***caractérisé en ce que* en ce que** le corps (4) est percé d'au moins un deuxième trou (8) dont une première extrémité débouche dans ledit volume creux, **en ce que** l'autre extrémité du premier trou (7) est reliée à l'embouchure d'entrée (5), respectivement à l'air libre, **en ce que** l'autre extrémité du deuxième trou (8) est reliée à l'air libre, respectivement à l'embouchure d'entrée (5'), afin qu'une pression, respectivement dépression, de fluide à l'embouchure d'entrée (5, 5') provoque une expansion de la membrane déformable (3) dans le volume creux délimité par le corps (4) **en ce que** l'indicateur comprend au moins un repère de pression porté par le corps (4), en regard d'une position d'expansion de la membrane déformable (3), permettant de déterminer en fonction de ladite position d'expansion de la membrane au moins deux plages de valeurs de pression, **en ce que** l'indicateur de pression est configuré de sorte que la membrane (3), au cours de son expansion, prenne appui contre une paroi du corps (2) portant l'au moins un repère de pression et **en ce que** l'indicateur de pression est configuré de sorte que la membrane (3) s'étale progressivement sur la paroi à mesure que la pression augmente.

2. Indicateur de pression selon la revendication précédente, où le corps (4) comprend au moins deux repères de pression chacun en regard d'une position d'expansion de la membrane déformable (3), permettant de déterminer en fonction de ladite position d'expansion de la membrane au moins trois plage de valeurs de pression.

3. Indicateur de pression selon l'une quelconque des revendications précédentes, où l'expansion (E) de la membrane déformable (3) pour la plage de pression est au moins égale à 1 mm, comprenant une unique membrane (3) déformable, la membrane (3) étant élastique et où la membrane (3) est monolithique.

4. Indicateur de pression selon l'une quelconque des revendications précédentes, configuré de sorte que lorsque la pression atteint une pression donnée, l'expansion de la membrane (3) provoque une mise en contact de la membrane (3) avec la paroi, puis lorsque la pression augmente, l'expansion de la membrane (3) entraîne l'étalement de la membrane (3) sur la paroi.

5. Indicateur de pression selon l'une quelconque des revendications précédentes, configuré de sorte que l'étalement de la membrane (3) sur la paroi est fonction de la pression à mesurer où la paroi s'étend dans un plan sensiblement parallèle à un plan dans lequel s'étend la membrane (3) lorsqu'elle est au repos.

6. Indicateur de pression selon l'une quelconque des revendications précédentes, où l'indicateur comprend au moins deux repères de pression portés chacun par ladite paroi sur laquelle la membrane (3) prend appui au cours de son expansion, et chacun en regard d'une position d'expansion de la membrane déformable (3) et configurés pour permettre de déterminer en fonction de ladite position d'expansion de la membrane au moins trois plage de valeurs de pression et où les repères de pression correspondent chacun à un étalement de la membrane (3) sur la paroi.

7. Indicateur de pression selon l'une quelconque des revendications précédentes, dans lequel la paroi est configurée de sorte à empêcher un utilisateur de visualiser la membrane (3) lorsque la membrane (3) n'est pas au contact de la paroi et de sorte à permettre à un utilisateur de visualiser une portion au moins de la membrane (3) lorsque la membrane (3) est au contact de la paroi.

8. Indicateur de pression selon la revendication précédente, conformé de manière à permettre de visualiser uniquement la portion de la membrane (3) qui est au contact de la paroi et conformé de manière à permettre de visualiser toute la portion de la membrane (3) qui est au contact de la paroi.

9. Indicateur de pression selon l'une quelconque des revendications précédentes, comprenant au moins trois repères (91-95) portés par ladite paroi sur laquelle la membrane (3) prend appui au cours de son expansion, et disposés de sorte à identifier au moins trois positions d'étalement de la membrane (3), et dans lequel la membrane s'étale sous forme de disque sur la paroi.

10. Indicateur de pression selon l'une quelconque des trois revendications précédentes, dans lequel la paroi est translucide.

11. Indicateur de pression selon l'une quelconque des revendications précédentes, où la membrane déformable (3) est réalisée en matériau thermoplastique élastomère type TPS-SEBS ou type SBS.et où la plage de pression est comprise entre 0-150 cm/H20 et où la membrane déformable (3) présente une dureté shore A de 0 et une épaisseur comprise entre 0.3 et 0,8 mm et de préférence dans lequel la plage de pression est 0-120 cm/H20 et où la membrane déformable (3) présente une dureté shore A de 0 et une épaisseur de 0,7 mm.

12. Indicateur de pression selon l'une quelconque des revendications précédentes, où la membrane déformable (3) comprend, au niveau du contour fermé (6), au moins un bourrelet, respectivement une gorge, coopérant avec une gorge, respectivement un bourrelet, pratiqué dans le socle (2) ou comprend au niveau du contour fermé (6), une alternance de bourrelets et de gorges, coopérant avec une alternance respectivement correspondante de gorges et de bourrelets pratiquée dans le socle (2).

13. Indicateur de pression selon l'une quelconque des revendications précédentes, où le matériau de la membrane déformable (3) ou la paroi, comprend un composé piezochrome, ou un composé tribochrome, ou un composé thermochrome.

14. Indicateur de pression selon l'une quelconque des revendications précédentes, où le corps (4) comprend encore au moins une lentille déformante (10, 11), afin de modifier la vision de la membrane déformable (3) en expansion et où la lentille déformante est disposée avec son axe optique parallèle à l'axe principal d'expansion.

15. Indicateur de pression selon l'une quelconque des revendications précédentes, où la paroi est dans une matière translucide et non transparente de sorte à empêcher l'utilisateur de visualiser les parties de membrane (3) qui ne sont pas au contact de la paroi.

16. Utilisation d'un indicateur de pression selon l'une quelconque des revendications précédentes pour surveiller une pression dans un coussinet (12) d'appareil médical, tel le coussinet gonflable (12) d'un masque laryngé (13).

17. Dispositif médical comportant un indicateur selon l'une quelconque des revendications 1 à 15 et un coussinet gonflable (12) associé à l'indicateur de manière à surveiller la pression du coussinet (12), et dans lequel le dispositif formant un masque laryngé (13) ou dans lequel l'un parmi le premier (7) et le deuxième (8) trou est en communication fluidique avec un orifice distal d'une aiguille de péridurale.

## Patentansprüche

1. Druckanzeiger, umfassend einen Sockel (2), eine entlang einer geschlossenen Kontur (6) fluiddicht am Sockel (2) befestigte verformbare Membran (3) und einen fest mit dem Sockel (2) verbundenen, ein Hohlvolumen begrenzenden Körper (4), der die verformbare Membran (3) auf der dem Sockel (2) gegenüberliegenden Seite unter Umschließen mindestens der geschlossenen Kontur (6) bedeckt, mindestens eine Einlassöffnung (5, 5') für ein Fluid, dessen Druck in einem Druckbereich gemessen werden soll, wobei die verformbare Membran (3) so ist, dass ihre Dehnung (E) bei dem Druckbereich ausreichend ist, um mit bloßem Auge erkennbar zu sein und eine direkte Anzeige zu ermöglichen, die den Druck anzeigt, wobei der Sockel (2) mit mindestens einem ersten Loch (7) durchbohrt ist, von dem ein erstes Ende zwischen der verformbaren Membran (3) und dem Sockel (2) in der geschlossenen Kontur (6) mündet, ***dadurch gekennzeichnet,*** dadurch dass der Körper (4) mit mindestens einem zweiten Loch (8) durchbohrt ist, von dem ein erstes Ende im Hohlvolumen mündet, dadurch, dass das andere Ende des ersten Lochs (7) mit der Einlassöffnung (5) beziehungsweise mit dem Freien verbunden ist, dadurch, dass das andere Ende des zweiten Lochs (8) mit dem Freien beziehungsweise mit der Einlassöffnung (5') verbunden ist, damit ein Fluiddruck beziehungsweise -unterdruck an der Einlassöffnung (5, 5') eine Dehnung der verformbaren Membran (3) in dem vom Körper (4) begrenzten Hohlvolumen bewirkt, dadurch, dass der Anzeiger mindestens eine vom Körper (4) getragene, einer Dehnungsstellung der verformbaren Membran (3) zugewandte Druckmarkierung umfasst, die es ermöglicht, abhängig von der Dehnungsstellung der Membran mindestens zwei Druckwertebereiche zu bestimmen, dadurch, dass der Druckanzeiger so konfiguriert ist, dass die Membran (3) im Laufe ihrer Dehnung an einer Wand des Körpers (2) in Anlage geht, die die mindestens eine Druckmarkierung trägt, und dadurch, dass der Druckanzeiger so konfiguriert ist, dass sich die Membran (3) mit steigendem Druck zunehmend an der Wand ausbreitet.

2. Druckanzeiger nach dem vorstehenden Anspruch, wobei der Körper (4) mindestens zwei Druckmarkierungen umfasst, jede einer Dehnungsstellung der verformbaren Membran (3) zugewandt, die es ermöglichen, abhängig von der Dehnungsstellung der Membran mindestens drei Druckwertebereiche zu bestimmen.

3. Druckanzeiger nach einem der vorstehenden Ansprüche, wobei die Dehnung (E) der verformbaren Membran (3) bei dem Druckbereich mindestens gleich 1 mm beträgt, umfassend eine einzige verformbare Membran (3), wobei die Membran (3) elastisch ist, und wobei die Membran (3) monolithisch ist.

4. Druckanzeiger nach einem der vorstehenden Ansprüche, der so konfiguriert ist, dass wenn der Druck einen gegebenen Druck erreicht, die Dehnung der Membran (3) ein Inkontaktbringen der Membran (3) mit der Wand bewirkt, und anschließend, wenn der Druck steigt, die Dehnung der Membran (3) das Ausbreiten der Membran (3) an der Wand nach sich zieht.

5. Druckanzeiger nach einem der vorstehenden Ansprüche, der so konfiguriert ist, dass das Ausbreiten der Membran (3) an der Wand von dem Druck abhängig ist, der gemessen werden soll, wobei sich die Wand in einer Ebene erstreckt, die im Wesentlichen zu einer Ebene parallel ist, in der sich die Membran (3) erstreckt, wenn sie sich im Ruhezustand befindet.

6. Druckanzeiger nach einem der vorstehenden Ansprüche, wobei der Anzeiger mindestens zwei Druckmarkierungen umfasst, die jede von der Wand getragen werden, an der die Membran (3) im Laufe ihrer Dehnung in Anlage geht, und jede einer Dehnungsstellung der verformbaren Membran (3) zugewandt und dafür konfiguriert sind, zu ermöglichen, abhängig von der Dehnungsstellung der Membran mindestens drei Druckwertebereiche zu bestimmen, und wobei die Druckmarkierungen jede einer Ausbreitung der Membran (3) an der Wand entsprechen.

7. Druckanzeiger nach einem der vorstehenden Ansprüche, wobei die Wand so konfiguriert ist, dass verhindert wird, dass ein Benutzer die Membran (3) sieht, wenn sich die Membran (3) nicht mit der Wand in Kontakt befindet, und so, dass es einem Benutzer ermöglicht wird, mindestens einen Abschnitt der Membran (3) zu sehen, wenn sich die Membran (3) mit der Wand in Kontakt befindet.

8. Druckanzeiger nach dem vorstehenden Anspruch, der so ausgestaltet ist, dass er es ermöglicht, nur den Abschnitt der Membran (3) zu sehen, der sich mit der Wand in Kontakt befindet, und so ausgestaltet ist, dass er es ermöglicht, den ganzen Abschnitt der Membran (3) zu sehen, der sich mit der Wand in Kontakt befindet.

9. Druckanzeiger nach einem der vorstehenden Ansprüche, der mindestens drei Markierungen (91-95) umfasst, die von der Wand getragen werden, an der die Membran (3) im Laufe ihrer Dehnung in Anlage geht, und die so angeordnet sind, dass mindestens drei Ausbreitungsstellungen der Membran (3) identifiziert werden, und wobei sich die Membran scheibenförmig an der Wand ausbreitet.

10. Druckanzeiger nach einem der drei vorstehenden Ansprüche, wobei die Wand durchscheinend ist.

11. Druckanzeiger nach einem der vorstehenden Ansprüche, wobei die verformbare Membran (3) aus thermoplastischem Elastomermaterial vom Typ TPS-SEBS oder Typ SBS ausgeführt ist, und wobei der Druckbereich im Bereich zwischen 0-150 cm/H20 liegt, und wobei die verformbare Membran (3) eine Shore-Härte A von 0 und eine Dicke im Bereich zwischen 0,3 und 0,8 mm aufweist, und wobei vorzugsweise der Druckbereich 0-120 cm/H20 beträgt und wobei die verformbare Membran (3) eine Shore-Härte A von 0 und eine Dicke von 0,7 mm aufweist.

12. Druckanzeiger nach einem der vorstehenden Ansprüche, wobei die verformbare Membran (3) im Bereich der geschlossenen Kontur (6) mindestens eine Wulst beziehungsweise eine Nut umfasst, die mit einer im Sockel (2) ausgeführten Nut beziehungsweise einer Wulst zusammenwirkt, oder im Bereich der geschlossenen Kontur (6) eine Abfolge von Wulsten und Nuten umfasst, die mit einer jeweils entsprechenden, im Sockel (2) ausgeführten Abfolge von Nuten und Wulsten zusammenwirkt.

13. Druckanzeiger nach einem der vorstehenden Ansprüche, wobei das Material der verformbaren Membran (3) oder der Wand eine piezochrome Verbindung oder eine tribochrome Verbindung oder eine thermochrome Verbindung umfasst.

14. Druckanzeiger nach einem der vorstehenden Ansprüche, wobei der Körper (4) noch mindestens eine Verformungslinse (10, 11) umfasst, um die Sicht der verformbaren Membran (3) unter Dehnung zu modifizieren, und wobei die Verformungslinse mit ihrer optischen Achse parallel zur Hauptdehnungsachse angeordnet ist.

15. Druckanzeiger nach einem der vorstehenden Ansprüche, wobei die Wand aus einem durchscheinenden, und nicht durchsichtigen Material ist, um zu verhindern, dass der Benutzer die Teile der Membran (3) sieht, die sich nicht mit der Wand in Kontakt befinden.

16. Verwendung eines Druckanzeigers nach einem der vorstehenden Ansprüche, um einen Druck in einem Polster (12) einer medizinischen Vorrichtung, wie etwa dem aufblasbaren Polster (12) einer Kehlkopfmaske (13), zu überwachen.

17. Medizinische Vorrichtung, die einen Anzeiger nach einem der Ansprüche 1 bis 15 und ein aufblasbares Polster (12) umfasst, das so mit dem Anzeiger verknüpft ist, dass Druck des Polsters (12) überwacht wird, und wobei die Vorrichtung eine Kehlkopfmaske (13) bildet, oder wobei eines aus dem ersten (7) und dem zweiten (8) Loch mit einer distalen Öffnung einer Epiduralnadel in Fluidkommunikation steht.

## Claims

1. Pressure indicator comprising a base (2), a deformable membrane (3) fixed to the base (2) sealed against fluids along a closed edge (6), and a body (4) secured to the base (2), delimiting a hollow volume covering the deformable membrane (3) on the side opposite the base (2), by comprising at least the closed edge (6), at least one inlet mouth (5, 5') for a fluid of which the pressure can be measured in a pressure range, wherein the deformable membrane (3) is such that the expansion (E) thereof for said pressure range is sufficient to be detectable to the naked eye and to make it possible for a direct display, indicating the pressure, wherein the base (2) is bored with at least one first hole (7) of which a first end opens between the deformable membrane (3) and the base (2) in the closed edge (6), **characterised in that** the body (4) is bored with at least one second hold (8) of which a first end opens into said hollow volume, **in that** the other end of the first hole (7) is connected to the inlet mouth (5), respectively to the open air, **in that** the other end of the second hole (8) is connected to the open air, respectively to the inlet mouth (5), such that a pressure, respectively depression, of fluid to the inlet mouth (5, 5') causes an expansion of the deformable membrane (3) in the hollow volume delimited by the body (4) **in that** the indicator comprises at least one pressure marker carried by the body (4), facing an expansion position of the deformable membrane (3), making it possible to determine, according to said expansion position of the membrane, two ranges of pressure values, **in that** the pressure indicator is configured such that the membrane (3), during the expansion thereof, bears against a wall of the body (2) carrying the at least one pressure marker and **in that** the pressure indicator is configured such that the membrane (3) progressively spread over the wall as the pressure increases.

2. Pressure indicator according to the preceding claim, where the body (4) comprises at least two pressure markers, each facing an expansion position of the deformable membrane (3), making it possible to determine, according to said expansion position of the membrane, at least three ranges of pressure values.

3. Pressure indicator according to any one of the preceding claims, where the expansion (E) of the deformable membrane (3) for the pressure range is at least equal to 1mm, comprising one single deformable membrane (3), the membrane (3) being elastic and where the membrane (3) is monolithic.

4. Pressure indicator according to any one of the preceding claims, configured such that the pressure reaches a given pressure, the expansion of the membrane (3) causes a putting into contact of the membrane (3) with the wall, then when the pressure increases, the expansion of the membrane (3) leads to the spreading of the membrane (3) over the wall.

5. Pressure indicator according to any one of the preceding claims, configured such that the spreading of the membrane (3) over the wall according to the pressure to be measured where the wall extends into a plane, substantially parallel to a plane wherein the membrane (3) extends when it is idle.

6. Pressure indicator according to any one of the preceding claims, where the indicator comprises at least two pressure markers each carried by said wall on which the membrane (3) bears during the expansion thereof, and each facing an expansion position of the deformable membrane (3) and configured to make it possible to determine, according to said expansion position of the membrane at least three ranges of pressure values and where the pressure markers each correspond to a spreading of the membrane (3) over the wall.

7. Pressure indicator according to any one of the preceding claims, wherein the wall is configured so as to prevent a user from viewing the membrane (3) when the membrane (3) is not in contact with the wall and so as to make it possible for a user to view at least one portion of the membrane (3) when the membrane (3) is in contact with the wall.

8. Pressure indicator according to the preceding claim, shaped to as to make it possible to only view the portion of the membrane (3) which is in contact with the wall and shaped so as to make it possible to view the whole portion of the membrane (3) which is in contact with the wall.

9. Pressure indicator according to any one of the preceding claims, comprising at least three markers (91-95) carried by said wall on which the membrane (3) bears during the expansion thereof, and arranged so as to identify at least three positions of spreading the membrane (3), and wherein the membrane is spread in the form of a disc over the wall.

10. Pressure indicator according to any one of the three preceding claims, wherein the wall is translucid.

11. Pressure indicator according to any one of the preceding claims, where the deformable membrane (3) is made of TPS-SEBS type or SBS type elastomer thermoplastic material, and where the pressure range is between 0-150cm/H20 and where the deformable membrane (3) has a Shore hardness A of 0 and a thickness of between 0.3 and 0.8mm, and preferably wherein the pressure range is 0-120cm/H20 and where the deformable membrane (3) has a Short hardness A of 0 and a thickness of 0.7mm.

12. Pressure indicator according to any one of the preceding claims, where the deformable membrane (3) comprises, at the level of the closed edge (6), at least one protrusion, respectively a recess, cooperating with a recess, respectively a protrusion, made in the base (2) or comprises at the level of the closed edge (6), an alternation of protrusions and recesses, cooperating with a respectively corresponding alternation of recesses and of protrusions made in the base (2).

13. Pressure indicator according to any one of the preceding claims, where the material of the deformable membrane (3) or the wall, comprises a piezochromic compound, or a tribochromic compound, or a thermochromic compound.

14. Pressure indicator according to any one of the preceding claims, where the body (4) also comprises at least one deforming lens (10, 11), in order to modify the vision of the expanding deformable membrane (3) and where the deforming lens is arranged with the optical axis thereof, parallel to the main expansion axis.

15. Pressure indicator according to any one of the preceding claims, where the wall is made of a translucid and non-transparent material so as to prevent the user from viewing the membrane (3) portions which are not in contact with the wall.

16. Use of a pressure indicator according to any one of the preceding claims, to monitor a pressure in a medical appliance pad (12), such as the inflatable pad (12) of a laryngeal mask (13).

17. Medical device comprising an indicator according to any one of claims 1 to 15, and an inflatable pad (12) associated with an indicator so as to monitor the pressure of the pad (12), and wherein the device forming a laryngeal mask (13) or wherein one from among the first (7) and the second (8) hole is in fluidic communication with a distal orifice of an epidural needle.
